# EUROPÄISCHE PATENTSCHRIFT

(11) **EP 0 601 483 B1**
(45) Veröffentlichungstag und Bekanntmachung des Hinweises auf die Patenterteilung: **27.09.1995**
(21) Anmeldenummer: 93119438.5
(22) Anmeldetag: 02.12.1993
(51) Int. Cl.: C09K 19/40, C08G 77/38

(54) **Pigmente mit vom Betrachtungswinkel abhängiger Farbigkeit, ihre Herstellung und Verwendung**
Pigments with colour dependant on viewing angle, it's production and use
Pigments avec coloration fonction de l'angle d'observation, sa production et son utilisation

(30) Priorität: 03.12.1992 DE 4240743
(43) Veröffentlichungstag der Anmeldung: 15.06.1994
(73) Patentinhaber: Consortium für elektrochemische Industrie GmbH, D-81379 München (DE)
(72) Erfinder: Müller-Rees, Christoph, Dr., D-82049 Pullach (DE); Maurer, Robert, Dr., D-81371 München (DE); Stohrer, Jürgen, Dr., D-81479 München (DE); Kreuzer, Franz-Heinrich, Dr., D-82152 Martinsried (DE); Jung, Silvia, D-80799 München (DE); Csellich, Franz, D-82008 Unterhaching (DE)

(56) Entgegenhaltungen:
- EP-A- 0 358 208
- EP-A- 0 383 376
- MOLECULAR CRYSTALS AND LIQUID CRYSTALS, LETTERS Bd. 123, Nr. 1/4 , 1985 Seiten 347 - 353 D. MAKOW 'color gamut of liquid crystal polysiloxanes'
- CONFERENCE RECORD OF THE 1991 INTERNATIONAL DISPLAY RESEARCH CONFERENCE (CAT. NO.91CH3071-8), SAN DIEGO, CA, USA, 15-17 OCT. 1991, 57 - 59 Haberle N et al 'Right and left circular polarizing color filters made from crosslinkable cholesteric LC-silicones'

## Beschreibung

Die Erfindung betrifft Pigmente mit vom Betrachtungswinkel abhängiger Farbigkeit, ihre Herstellung und Verwendung.

Die im Stand der Technik am weitesten verbreiteten Pigmente bestehen aus Partikeln, die einen Teil des einfallenden Lichtes absorbieren und den Rest reflektieren. Der reflektierte Teil des Lichtes bestimmt den Farbeindruck.

Daneben gibt es Pigmente, die Ihre Farbigkeit Interferenzeffekten verdanken. Diese Pigmente sind als Perlglanzpigmente bekannt. Sie bestehen aus einem Trägermaterial, meist Glimmerplättchen, auf das dünne Schichten von Substanzen mit unterschiedlicher optischer Dichte aufgebracht sind. Diese Schichten bestehen häufig aus Schwermetallverbindungen (Ullmanns Encyclopädie der technischen Chemie, 4. Auflage, 1976, Band 18, S. 631 - 634). Die Anwendung von schwermetallhaltigen Perlglanzpigmenten ist aus Gründen der Umweltbelastung bei ihrer Verarbeitung und Entsorgung problematisch.

In der EP-A-383 376 ist die Verwendung von cholesterischen Flüssigkristallen an Stelle der Schwermetallverbindungen zur Beschichtung von Glimmerplättchen und anderen organischen und anorganischen Trägermaterialien beschrieben. Die Verwendung der cholesterischen Flüssigkristalle vermeidet zwar den Nachteil einer schwermetallhaltigen Beschichtung, leidet jedoch unter der Tatsache, daß die verwendeten Trägermaterialien selbst häufig schwermetallhaltig sind.

Für die Herstellung der genannten Pigmente sind zudem gewisse Anforderungen an Trägermaterial und Beschichtung zu stellen. So darf das Trägermaterial eine bestimmte Dicke nicht übersteigen, da sonst die Herstellung feinteiliger Pigmente erschwert wird. Bei der Verwendung von Glimmer oder Metallflittern sind die Trägermaterialien zwar sehr dünn, erfordern jedoch ein Coaten auf der Vorder- und Rückseite in genau einzuhaltenden Schichtdicken.

Aufgabe der Erfindung war es, Pigmente bereitzustellen, die die Nachteile des Stands der Technik vermeiden und neuartige Farbeffekte mit zirkular polarisiertem Licht ermöglichen.

Die Aufgabe wird gelöst durch Pigmente einer Dicke von 1 bis 100 µm und einem Durchmesser von 1 bis 10.000 µm mit vom Betrachtungswinkel abhängiger Farbigkeit, die dadurch gekennzeichnet sind, daß sie aus orientierten dreidimensional vernetzten Substanzen flüssigkristalliner Struktur mit chiraler Phase sowie gegebenenfalls weiteren Farbstoffen und Pigmenten bestehen, wobei die gegebenenfalls vorhandenen weiteren Farbstoffe und Pigmente nicht als Träger für die orientierten dreidimensional vernetzten flüssigkristallinen Substanzen mit chiraler Phase dienen.

Unter Farbigkeit ist im Sinne der Erfindung nicht nur der mit dem menschlichen Auge wahrnehmbare Farbeindruck des Wellenlängenbereichs des sichtbaren Lichtes zu verstehen, sondern ebenso der mit dem menschlichen Auge nicht wahrnehmbare aber mittels bekannter Geräte wie UV- und IR-Spektrometer meßbare Farbeindruck der benachbarten UV- bzw IR- Wellenlängenbereiche.

In einer Ausführungsform bestehen die erfindungsgemäßen Pigmente ausschließlich aus einer Interferenzschicht, wobei diese Interferenzschicht aus orientierten dreidimensional vernetzten flüssigkristallinen Substanzen mit chiraler Phase besteht. Die Farbigkeit dieser Pigmente beruht somit ausschließlich auf einem Interferenzeffekt. Das von diesen Pigmenten reflektierte Licht ist zirkular polarisiert.

In einer anderen Ausführungsform enthalten die erfindungsgemäßen Pigmente zusätzlich zu den orientierten dreidimensional vernetzten flüssigkristallinen Substanzen mit chiraler Phase andere Farbstoffe oder Pigmente. Geeignete Farbstoffe sind in den nichtpolymerisierten Ausgangssubstanzen zur Herstellung der erfindungsgemäßen Pigmente löslich. Geeignete zusätzliche Pigmente sind mit den nichtpolymerisierten Ausgangssubstanzen zur Herstellung der erfindungsgemäßen Pigmente mischbar. In dieser Ausführungsform sind die winkelabhängigen Farbeffekte der erfindungsgemäßen Pigmente mit zusätzlichen Farbeffekten der bekannten Pigmente und Farbstoffe kombiniert. Bevorzugt geeignet sind schwermetallfreie zusätzliche Pigmente. Enthalten die erfindungsgemäßen Pigmente beispielsweise Gasruß, wird der nicht reflektierte Anteil des einfallenden Lichtes im Pigment absorbiert. Der gewünschte Farbeindruck der Pigmente wird nicht durch eine eventuelle Reflexion des Untergrundes beeinträchtigt.

Die erfindungsgemäßen Pigmente enthalten kein Trägermaterial, auf welches die orientierten dreidimensional vernetzten flüssigkristallinen Substanzen mit chiraler Phase aufgetragen sind.

Die erfindungsgemäßen Pigmente lassen sich beliebig miteinander mischen. Dadurch ist es erstmals möglich, beliebige definierte Farbeffekte, die mit dem Betrachtungswinkel variieren, mittels flüssigkristalliner Substanzen durch einfaches Abmischen herzustellen und so mittels flüssigkristalliner Substanzen andere Farben als reine Spektralfarben zu erzeugen. So lassen sich durch das Mischen erfindungsgemäßer Pigmente beispielsweise erstmals Purpurfarbtöne mittels flüssigkristalliner Substanzen herstellen.

Die erfindungsgemäßen Pigmente sind dadurch erhältlich, daß dreidimensional vernetzbare flüssigkristalline Substanzen mit chiraler Phase gegebenenfalls nach Zumischen weiterer Farbstoffe und/oder Pigmente orientiert werden, dreidimensional vernetzt werden und auf die erwünschte Korngröße zerkleinert werden.

Bevorzugt werden die dreidimensional vernetzbaren flüssigkristallinen Substanzen mit chiraler Phase auf eine Unterlage aufgebracht, auf dieser Unterlage vernetzt und nach dem Vernetzen von der Unterlage abgelöst.

Flüssigkristalline Substanzen, die als Ausgangssubstanzen für die Herstellung der erfindungsgemäßen Pigmente geeignet sind, besitzen eine verdrillte Struktur mit einer Ganghöhe, die einer Wellenlänge des Lichtes im Bereich von UV bis IR entspricht. Diese Struktur findet sich beispielsweise bei cholesterischen Flüssigkristallen. Cholesterische Flüssigkristalle, oder allgemein flüssigkristalline Substanzen mit chiraler Phase, die eine verdrillte Struktur mit einer gewünschten Ganghöhe besitzen, können aus nematischen, smektischen oder diskotischen Strukturen erhalten werden, indem man ihnen eine chirale Substanz zusetzt. Art und Anteil der chiralen Substanz bestimmen die Ganghöhe der verdrillten Struktur und damit die Wellenlänge des reflektierten Lichtes. Die Verdrillung der Struktur kann sowohl links- als auch rechtshändig sein. Die Ausgangssubstanzen müssen zudem polymerisierbare, polykondensierbare oder einer Polyaddition zugängliche Gruppen, von denen zumindest ein Teil in Form di-, tri- oder höherfunktioneller Bausteine vorliegt, enthalten. Beispiele für solche Gruppen sind Methacryloxy- und Acryloxygruppen.

Geeignete Materialien und ihre Herstellung sind beispielsweise in DE-C2-3,604,757, in EP-A2-358,208, in EP-A-0 066 137 (entspricht US 4,388,453) oder in der in D.J. Broer et al in 14. Int. Liquid Conf., Abstracts II, 921 (1992) genannten Literatur beschrieben.

Bevorzugt geeignet sind dreidimensional vernetzbare Polyorganosiloxane nach EP-A-358,208.

Als Ausgangsstoffe zur Herstellung der erfindungsgemäßen Pigmente können jedoch grundsätzlich alle cholesterischen Flüssigkristalle dienen. Es kann eine Art von cholesterischem Flüssigkristall, es kann aber auch ein Gemisch aus mindestens zwei dieser Flüssigkristalle eingesetzt werden; es kann ein Farbstoff, es können auch Gemische aus mindestens zwei Farbstoffen eingesetzt werden.

Der einzusetzende Farbstoff ist in einer bevorzugten Ausführungsform ein Pigment. Der im erfindungsgemäßen Verfahren einzusetzende Farbstoff ist in einer weiteren bevorzugten Ausführungsform im eingesetzten Flüssigkristall(gemisch) löslich. Vorzugsweise wird kein Gemisch von mehreren cholesterischen flüssigkristallinen Substanzen, sondern eine einzelne reine cholesterische flüssigkristalline Substanz im erfindungsgemäßen Verfahren eingesetzt.

Das Zumischen der Pigmente und/oder Farbstoffe zu den übrigen Ausgangssubstanzen erfolgt in üblicher Art und Weise beispielsweise durch Einrühren. Das Zumischen der Farbstoffe und/oder Pigmente bewirkt im erfindungsgemäßen Pigment eine Kombination der winkelabhängigen Farbeffekte der flüssigkristallinen Substanzen mit dem oder den bekannten Farbeffekten der jeweiligen zugemischten Stoffe. Das Zumischen dieser Stoffe ändert jedoch nichts an den weiteren Verfahrensschritten zur Herstellung der erfindungsgemäßen Pigmente.

Eine jeweils gewünschte Pigmentfarbe kann auch dadurch erhalten werden, daß definierte Flüssigkristall-Grundmischungen in geeigneten Mengenverhältnissen gemischt werden. Auch in diesem Falle ändern sich die weiteren Verfahrensschritte zur Herstellung der erfindungsgemäßen Pigmente nicht. Die weitere Beschreibung des Herstellungsverfahrens gilt daher für alle Varianten der erfindungsmäßen Pigmente.

Flüssigkristalle mit verdrillten Phasen bilden ihre optischen Eigenschaften erst dann aus, wenn die einzelnen Moleküle in Schichten angeordnet sind und innerhalb einer Schicht einheitlich geordnet sind. Die Moleküle ändern dabei von Schicht zu Schicht ihre Vorzugsrichtung, so daß schraubenförmige Strukturen entstehen. Um dies zu erreichen, werden die Moleküle mittels bekannter Methoden wie beispielsweise durch Orientierungsschichten oder elektrische oder magnetische Felder orientiert. Soche Methoden sind beispielsweise aus den folgenden Literaturstellen bekannt: CA113 (22), 201523y; CA113 (14), 124523u; CA112 (18), 169216s; CA112 (16), 149138q; CA112 (4), 21552c; CA111 (16), 144258y; CA111 (4), 24780r;
Bei der Herstellung der erfindungsgemäßen Pigmente werden die genannten Ausgangssubstanzen in bekannter Weise orientiert. Dies kann beispielsweise durch Aufrakeln auf eine Metall-, Kunststoff- oder Glasunterlage geschehen. Diese Unterlage kann gegebenenfalls mit einer Orientierungsschicht z.B. aus Polyimid oder Polyvinylalkohol versehen sein. Sie kann zu diesem Zweck auch silanisiert sein. Es ist jedoch ebenso möglich, die Ausgangssubstanz zwischen zwei Folien zu scheren. Bevorzugt werden eine oder zwei Polyethylenterephthalat Folien verwendet.

Das Aufrakeln flüssigkristalliner Polyorganosiloxane auf eine Folie ist beispielsweise aus EP-A-358,208 bekannt.

Die Vernetzung der orientierten flüssigkristallinen Substanzen erfolgt, wie für das jeweilige Material aus dem Stand der Technik bekannt. So können beispielsweise flüssigkristalline Polyorganosiloxane nach dem in der EP-A-66 137 beschriebenen Verfahren thermisch vernetzt werden. Die in der EP-A-358 208 beschriebenen flüssigkristallinen Polyorganosiloxane lassen sich photochemisch beispielsweise durch Bestrahlen mit UV-Licht dreidimensional vernetzen. Einen Überblick über Verfahren, orientierte Ausgangsstoffe photochemisch zu vernetzen, findet sich bei C.G. Roffey, Photopolymerisation of Surface Coatings, (1982) John Willey & Sons, Chichester, S. 137-208.

Die vernetzten orientierten flüssigkristallinen Substanzen mit chiraler Phase werden gegebenenfalls von der Unterlage abgelöst. Bei Verwendung einer Folie als Unterlage kann die mechanische Abtrennung der spröden vernetzten Flüssigkristalle von der Unterlage z.B. dadurch erfolgen, daß die Unterlage über eine Umlenkrolle mit kleinem Durchmesser geführt wird. Dadurch blättert das vernetzte Material von der Folie ab. Jede andere Methode, mit der sich das polymerisierte Material von der Unterlage entfernen läßt, ist jedoch ebenso geeignet.

Das orientierte dreidimensional vernetzte trägerlose flüssigkristalline Material wird auf eine jeweils erwünschte Korngröße zerkleinert. Dies kann z. B. durch Mahlen beispielsweise in Universalmühlen erfolgen. Je nach der erwünschten Anwendung der Pigmente können Korngrößen mit einem Durchmesser von etwa 10 mm bis zu einem µm hergestellt werden. Bevorzugt haben die Pigmente eine Korngröße zwischen 5 mm und 5 µm. Die Pigmente haben eine Dicke zwischen 1 und 100 µm, vorzugsweise 5 bis 50 µm.

Das Mahlgut kann anschließend zur Verengung der Korngrößenverteilung beispielsweise durch einen Siebprozeß klassiert werden.

Die erfindungsgemäßen Pigmente eignen sich zum Färben von unterschiedlichsten Materialien wie Lacken, Kunststoffen, Faserrohstoffen, Kosmetika oder von Druckfarben aller Art wie z.B. Siebdruckfarben. Die Pigmente werden dazu wie bekannte Pigmente in das jeweilige Material eingearbeitet. Die so hergestellten jeweiligen pigmenthaltigen Zusammensetzungen zeigen die gleichen farblichen Eigenschaften, die für die Pigmente selbst beschrieben wurden.

Die Erfindung betrifft ebenfalls Zusammensetzungen, die mindestens ein erfindungsgemäßes Pigment enthalten. Insbesondere handelt es sich dabei um Zusammensetzungen, die neben den erfindungsgemäßen Pigmenten noch mindestens eine Substanz aus der Gruppe Phenolharze, Aminharze, Alkydharze, Polyvinylacetatharze, Epoxidharze, Polyurethanharze, Polyethylenharze, Chlorkautschukharze, Cyclokautschukharze, chloriertes Polypropylen, Ketonharze, Acrylatharze, Melaminharze, Harnstoff-Formaldehyd-Harze, Phenol-Formaldehyd-Harze, oder mindestens eine Substanz aus der Gruppe Acrylnitril-Butadien-Styrol-Copolymere, Celluloseacetat, Celluloseacetobutyrat, Celluloseacetopropionat, Cellulosenitrat, Cellulosepropionat, Kunsthorn, Polyamid, Polycarbonat, Polyethylen, Polybutylenterephthalat, Polymethylmethacrylat, Polypropylen, Polystyrol, Polytetrafluorethylen, Polyvinylchlorid, Polyvinylidenchlorid, Polyurethan, Styrol-Acrylnitril-Copolymere, ungesättigte Polyesterharze enthalten.

Alle vorgenannten Zusammensetzungen ermöglichen vor allem in Verbindung mit glatten gekrümmten Flächen völlig neuartige Farbeffekte. Diese Flächen erscheinen in sich unterschiedlich gefärbt und diese unterschiedliche Färbung verändert sich noch je nach Standpunkt des Beobachters.

Erfindungsgemäße Zusammensetzungen enthaltend mindestens eine Substanz aus der Gruppe Phenolharze, Aminharze, Alkydharze, Polyvinylacetatharze, Epoxidharze, Polyurethanharze, Polyethylenharze, Chlorkautschukharze, Cyclokautschukharze, chloriertes Polypropylen, Ketonharze, Acrylatharze, Melaminharze, Harnstoff-Formaldehyd-Harze, Phenol-Formaldehyd-Harze, sowie etwa 10 Gew.-% (bezogen auf das Gesamtgewicht) erfindungsgemäße Pigmente zeigen nach Auftragen auf ein schwarzes Blech eine hohe Farbenbrillanz und eine vom Betrachtungswinkel abhängige variierende Farberscheinung. Das reflektierte Licht ist dabei zirkular polarisiert. Selbst wenn das in die Zusammensetzung eingearbeitete flüssigkristalline Polyorganosiloxan-Pigment in Korngrößen kleiner als 25 µm verwendet wird, bleibt die hohe Farbenbrillanz und die vom Betrachtungswinkel abhängige variierende Farberscheinung erhalten.

Zusammensetzungen, die mindestens ein erfindungsgemäßes Pigment sowie eine Substanz aus der Gruppe Phenolharze, Aminharze, Alkydharze, Polyvinylacetatharze, Epoxidharze, Polyurethanharze, Polyethylenharze, Chlorkautschukharze, Cyclokautschukharze, chloriertes Polypropylen, Ketonharze, Acrylatharze, Melaminharze, Harnstoff-Formaldehyd-Harze, Phenol-Formaldehyd-Harze enthalten, eignen sich besonders zur Beschichtung von metallischen Oberflächen. Werden solche Zusammensetzungen beispielsweise für die Lackierung von Kraftfahrzeugen eingesetzt, so erscheint ein derartig lackiertes Fahrzeug dem Beobachter im Vorbeifahren in verschiedenen Farben. Diese Farben lassen sich durch entsprechende Auswahl der eingesetzten Pigmente beliebig einstellen.

Die erfindungsgemäßen Pigmente eignen sich weiterhin als Sicherheitsmarkierung. So können beispielsweise Lacke und Folien, die erfindungsgemäße Pigmente mit einer Reflexion im UV- oder IR-Bereich enthalten, als für das menschliche Auge unsichtbare Markierungen und Sicherheitsmarken verwendet werden. Sie können anhand der Polarisation oder der Winkelabhängigkeit des reflektierten oder transmittierten Lichtes detektiert werden.

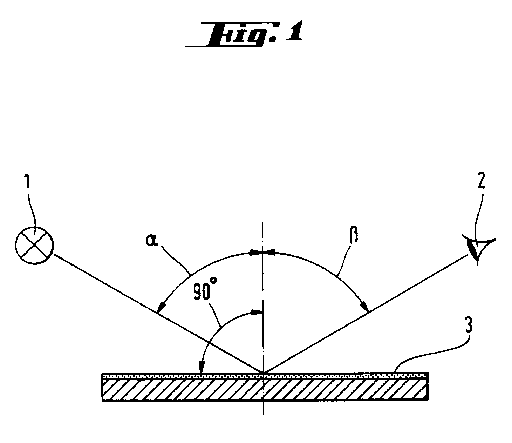

Fig. 1 zeigt die in Beispiel 7 beschriebene Betrachtung einer Oberfläche, die mit einem Lack, enthaltend erfindungsgemäße Pigmente, beschichtet ist. Dabei bedeutet 1 Lichtquelle; 2 Betrachter; 3 beschichtete Oberfläche; α Einfallswinkel, Winkel zwischen der Flächennormalen und der Lichtquelle; β Beobachtungswinkel, Winkel zwischen der Flächennormalen und dem Beobachter.

Die folgenden Beispiele dienen der weiteren Erläuterung der Erfindung.

### Beispiel 1:

### A: Polyorganosiloxane mit methacrylsäurehaltigen Seitenketten

Eine Lösung aus 233 g 4-(Prop-2-en-1-oxy)benzoesäurecholesterinester (erhältlich gemäß DE-A 31 10 048), 178 g 4-(Prop-2-en-1-oxy)benzoesäure(4-trimethylsiloxyphenyl)ester (erhältlich gemäß EP-A-358 208, Seite 9, Abschnitt C) und 56,9 g Tetramethylcyclotetrasiloxan in 400 ml Toluol wurde in Gegenwart von 24 mg Dicyclopentadienplatindichlorid 1 h und nach Zusatz einer Lösung von 1,2 g NaOH in 50 ml Ethanol weitere 7 h unter Rückfluß gekocht, um den Silylether zu spalten. Die Reaktionsmischung wurde auf 1/3 ihres Volumens im Rotationsverdampfer eingeengt, mit 7,5 g p-Toluolsulfonsäure und 154 g Methacrylsäureanhydrid versetzt und 1 h auf 100°C erwärmt. Nach dem Abdestillieren der flüchtigen Bestandteile wurde zweimal mit Methylenchlorid/Ethanol umgefällt.

Das Produkt hatte folgende physikalische und thermodynamische Daten: Glaspunkt: 14°C, Klärpunkt: 141°C.

### B Darstellung eines Pigments

4 g des Polyorganosiloxans, hergestellt wie in A beschrieben, wurden auf 70°C erwärmt und mit 0,11 g 2-Methyl-1-[4-(methylthio)phenyl]-2-morpholino-propanon-1 (erhältlich unter der Bezeichnung Irgacure 907 bei Ciba Marienberg GmbH, 6140 Bensheim 1) unter Umrühren homogen vermischt. Man erhielt eine rötlich schimmernde, zähflüssige LC-Masse. Das flüssigkristalline Material wurde bei 120°C mit Hilfe einer Rakel auf eine Polyethylenterephthalatfolie (Hoechst AG, Geschäftsbereich Folien, 6200 Wiesbaden 1) in Schichtdicken von 15 µm aufgetragen, wobei die Folie unter der fixierten Rakel mit einer Laufgeschwindigkeit von etwa 2 m/min fortbewegt wurde. Gleichzeitig erfolgte aufgrund des Schergefälles zwischen Rakel und Folie die Orientierung der flüssigkristallinen Moleküle, was durch eine rote Farberscheinung der Flüssigkristallschicht sichtbar wurde. Anschließend wurde diese Schicht mit Hilfe einer Quecksilberentladungslampe (80 W/cm) 5 Sekunden bestrahlt und so dreidimensional vernetzt. Der auf der Folie entstandene Film war im heißen und kalten Zustand klebfrei und spröde. Er hatte eine Reflexionswellenlänge von 560 nm. (Einfallswinkel und Beobachtungswinkel 45°, siehe Fig. 1). Die mechanische Abtrennung des auf diese Weise erhaltenen flüssigkristallinen Materials von der Unterlage wurde dadurch erreicht, daß die Folie über eine Umlenkrolle mit 10 cm Durchmesser geführt wurde und so das vernetzte Material vom Träger abblättert. Das Mahlen des vernetzten, substratfreien Materials wurde in einer Universalmühle durchgeführt. Durch 5 minütiges Mahlen der überwiegend in Blättchenform (Größe: einige Millimeter bis Zentimeter) anfallenden vernetzten Polyorganosiloxane entstand eine pulverförmige Fraktion. Das Mahlgut wurde dann zur Verengung der Korngrößenverteilung einem Siebprozeß unterworfen. Dazu wurden die gemahlenen Pigmente mittels eines Analysensiebes mit einer Maschenweite von 100 µm gesiebt.

### Beispiel 2:

### A Herstellung einer blauen Flüssigkristallmischung

Wie in Beispiel 1 beschrieben, wurden 6 g des Polyorganosiloxans hergestellt. Dieses wurde in 50 ml Toluol gelöst. Dieser Lösung wurden 2.6 g Methacrylsäurecholesterinester (hergestellt wie in De Visser et al., J. Polym. Sci., A 1(9), 1893 (1971) beschrieben) und 9 mg Aluminiumkupferon (erhältlich unter der Bezeichnung Q1301 von Wako Chemicals GmbH, 4040 Neuss) zugegeben. Das Toluol wurde dann unter Vakuum bei 70°C im Rotationsverdampfer entfernt. Es entstand eine zähflüssige LC-Masse mit folgenden physikalischen und thermodynamischen Daten: Glaspunkt: 4°C, Klärpunkt: 132°C.

### B Darstellung eines Pigments

4 g der LC-Masse, hergestellt wie unter A beschrieben, wur den auf 70°C erwärmt und mit 0.11 g 2-Methyl-1-(4-(methylthio)phenyl)-2-morpholino-Propanon-1 (erhältlich unter der Bezeichnung Irgacure 907 bei Ciba Marienberg GmbH, 6140 Bensheim 1) unter Umrühren homogen vermischt. Das flüssigkristalline Material wurde weiterverarbeitet wie unter B in Beispiel 1 beschrieben, wobei es mit einer Temperatur von 80°C auf der Folie aufgetragen und photochemisch vernetzt wurde. Der auf der Folie entstandene Film hatte eine Reflexionswellenlänge von 400 nm. Die Pigmente hatten eine intensiv blaue Farbe.

### Beispiel 3

### A Darstellung eines polymerisierbaren Monomeren:

### Methacryloxybenzoesäure(4-ethylphenyl)ester

Eine Lösung von 16,9 g 4-Trimethylsilyloxybenzoesäure(4-ethyl)phenylester (hergestellt analog der Vorschrift in EP-A-358 208, Seite 9, Abschnitt C) in 15 ml Toluol und 10 ml Ethanol wurde 1 Stunde unter Rückfluß gekocht und anschließend durch erhitzen auf 100°C für 60 min von flüchtigen Bestandteilen befreit. Die verbliebenen 13,3 g 4-Hydroxybenzoesäure(4-ethyl)phenylester wurden zusammen mit 30 g Methacrylsäureanhydrid und 1,2 g Toluolsulfonsäure in 15 ml Toluol gelöst und 1 h auf 100°C erwärmt. Nach dem Abkühlen wurde das Produkt mit Hexan ausgefällt und aus Ethanol umkristallisiert.

### B Herstellung einer roten Flüssigkristallmischung

6 g des Polyorganosiloxans, hergestellt wie in Beispiel 1 A wurden in 50 ml Toluol gelöst. Dieser Lösung wurden 1,5 g Methacryloxybenzoesäure(4-ethyl-phenyl)ester (hergestellt wie in Bsp.3 A) und 7,5 mg Aluminiumkupferon (erhältlich unter der Bezeichnung Q 1301 bei Wako Chemicals GmbH, 4040 Neuss) zugegeben. Das Toluol wurde dann unter Vakuum bei 70°C im Rotationsverdampfer entfernt.
Es entsteht eine zähflüssige LC-Masse mit folgenden physikalischen und thermodynamischen Daten: Glaspunkt: -2°C, Klärpunkt: 124°C.

### C Darstellung eines Pigments

Die so erhaltene Mischung wurde behandelt wie in Beispiel 2B beschrieben. Der auf der Folie entstandene Film hatte eine Reflexionswellenlänge von 630 nm. Man erhält Pigmente mit einer intensiv roten Farbe.

### Beispiel 4

### A Herstellung einer grünen Flüssigkristallmischung

2,8 g der roten Farbmischung (hergestellt wie in Beispiel 3 B beschrieben), 1,2 g der blauen Farbmischung (hergestellt wie in Beispiel 2 A beschrieben) und 0,11 g 2-Methyl- 1-[4-(methylthio)phenyl]-2-morpholino-propanon-1 (erhältlich unter der Bezeichnung Irgacure 907 bei Ciba Marienberg GmbH, 6140 Bensheim 1) wurden unter Umrühren homogen vermischt. Man erhält eine grünlich schimmernde, zähflüssige LC-Masse mit folgenden thermodynamischen Daten: Glaspunkt: 2°C, Klärpunkt: 128°C.

### B Darstellung eines Pigments

Die so erhaltene Mischung wurde wie in Beispiel 2B beschrieben weiterverarbeitet, indem sie bei einer Temperatur von 80°C auf eine Folie aufgetragen und photochemisch vernetzt wurde. Der auf der Folie entstandene Film hatte eine Reflexionswellenlänge von 530 nm. Man erhält Pigmente mit einer intensiv grünen Farbe.

### Beispiel 5

### A Herstellung einer grünen Flüssigkristallmischung

Aus 2 g 11-Methacryloxyundecansäurecholesterinester (hergestellt nach P.J.Shannon et al., Macromolecules, 1984, 17, 1873-1876;), 2 g 4-Methacryloxybutansäurecholesterinester (hergestellt nach P.J.Shannon et al., Macromolecules, 1984, 17, 1873-1876;) und 0.1 g Hexandioldiacrylat (erhältlich von Janssen Chimica, 4057 Brüggen 2) wurde durch Erwärmen auf 50°C und Rühren eine homogene, grün schillernde Mischung hergestellt.

### B Darstellung eines Pigments

Die so erhaltene Mischung wurde, wie in Beispiel 2 B beschrieben, zu Pigmenten weiterverarbeitet (Temperatur auf der Folie: 30°C). Man erhält Pigmente mit einer intensiv grünen Farbe.

### Beispiel 6

### Herstellung eines Pigments, enthaltend Gasruß

In 10 g einer Mischung, erhalten wie in Beispiel 4 A, wurden durch Rühren 0.2 g FW1(HCC)-Gasruß (Degussa, Frankfurt) homogen eingearbeitet. Die so erhaltene Mischung wurde, wie in Beispiel 2 B beschrieben, weiter verarbeitet. Man erhält Pigmente mit einer intensiv grünen Farbe auch auf weißem Untergrund.

### Beispiel 7

### Herstellung und Anwendung eines Lackes, enthaltend grüne Pigmente

1 g der in Beispiel 4 hergestellten Pigmentfraktion (Teilchengröße < 100 µm wurden 5 Minuten unter Rühren in 2 g Verdünner (Permacron Supercryl, Verdünnung 3054, Fa. Spies und Hecker, 5000 Köln) dispergiert. Diese Dispersion wurde in ein Gemisch aus 5 g Lack (Permacron MS Klarlack 8010, Spies und Hecker) und 2.5 g Härter (Permacron MS Spezial Härter 3368, Spies und Hecker) zugegeben. Die so erhaltene Lackformulierung wurde mit Hilfe einer Farbspritzpistole (Fa. Sata-Farbspritztechnik GmbH, Ludwigsburg) in Form feiner Tröpfchen auf ein mit schwarzer Farbe grundiertes Blech (Größe: 20x25 cm) gleichmäßig aufgesprüht. Nach dem Lackieren wurde 10 Minuten lang bei 80°C vorgetrocknet und als Schutzschicht ein Clearcoat (Lackformulierung wie beschrieben, aber ohne Pigmente) zweimal aufgetragen.

Das resultierende Blech zeigt eine grüne Farbe hoher Brillanz, wenn die Winkel α und β klein sind (Fig. 1). Mit zunehmenden Winkeln α und β geht die grüne Farbe kontinuierlich in eine blaue Farbe über. Bei gekrümmten Flächen und diffusen, nicht gerichtetem Licht sind somit je nach Betrachtungswinkel mehrere Farben zwischen grün und blau gleichzeitig sichtbar.

### Beispiel 8

### Herstellung und Anwendung eines Lackes, enthaltend Mischungen erfindungsgemäßer Pigmente

1 g blauer Pigmente, hergestellt wie in Beispiel 2 beschrieben, und 1 g roter Pigmente, hergestellt wie in Beispiel 3 beschrieben, wurden in 4 g Verdünner 5 Minuten unter Rühren dispergiert, wie in Beispiel 7 beschrieben weiter verarbeitet und auf ein schwarz grundiertes Blech aufgesprüht. Das Blech wurde wie in Beispiel 7 beschrieben mit einer Clearcoat-Schutzschicht versehen. Man beobachtet eine purpurne Farbe hoher Brillanz für kleine Werte von α und β, die bei zunehmenden Winkeln α und β kontinuierlich in einen türkisartigen Farbton übergeht.

### Beispiel 9:

### Bestimmung der Polarisation des reflektierten Lichtes.

Betrachtet man das nach Beispiel 4 beschichtete Blech durch einen Polarisator für linkszirkular-polarisiertes bzw. rechtszirkular-polarisiertes Licht, so ist die Reflexionsfarbe im ersten Fall sichtbar, während im zweiten Fall das Blech schwarz erscheint.

### Beispiel 10: Einarbeitung grüner Pigmente in Hart-PVC

90 Gewichtsteile Hart-PVC (erhältlich bei der Wacker-Chemie GmbH, München unter der Bezeichnung Vinnol H 70 F), 10 Gewichtsteile Phthalsäure-bis-(2-ethylhexylester) (Janssen Chimica, 4057 Brüggen 2), 2 Gewichtsteile Hostastab SnOS 661 (Hoechst AG, Frankfurt), 1 Teil teilverseiftes Esterwachs aus Montansäure (Wachs OP; Hoechst AG, Frankfurt) und 10 Gewichtsteile grüne Pigmente aus Beispiel 4 wurden zusammengegeben und mit einer Walze bei 150°C und einer Friktion von 1:1.1 10 min homogenisiert. Die entstandene Masse wurde bei 170°C in einer Presse zu Platten verarbeitet. Die Platten zeigen die gleichen grünblauen Farbeffekte wie das in den vorigen Beispielen beschriebene beschichtete Blech.

### Beispiel 11: Einarbeitung grüner Pigmente in Weich-PVC

100 Gewichtsteile Weich-PVC (erhältlich bei der Wacker-Chemie GmbH, München unter der Bezeichnung Vinnol P 70), 50 Gewichtsteile Phthalsäure-bis-(2-ethylhexylester), 1 Teil Barium-Zink- Stabilisator (erhältlich als Irgastab BZ 505 bei der Firma Ciba AG, Basel) und 7,5 Gewichtsteile der in Beispiel 4 hergestellten grünen Pigmente wurden zusammengegeben, homogenisiert und auf eine Glasplatte aufgegossen. Nach 10 minütigem Gelieren im Trockenschrank bei 180°C erhält man einen transparenten Film, der einen winkelabhängigen Farbeffekt wie in den vorigen Beispielen beschrieben aufweist.

## Patentansprüche

1. Pigmente einer Dicke von 1 bis 100 µm und einem Durchmesser von 1 bis 10000 µm mit vom Betrachtungswinkel abhängiger Farbigkeit, dadurch gekennzeichnet, daß sie aus orientierten dreidimensional vernetzten Substanzen mit flüssigkristalliner Struktur mit chiraler Phase sowie gegebenenfalls weiteren Farbstoffen und Pigmenten bestehen, wobei die gegebenenfalls vorhandenen weiteren Farbstoffe und Pigmente nicht als Träger für die orientierten dreidimensional vernetzten flüssigkristallinen Substanzen mit chiraler Phase dienen.

2. Pigmente nach Anspruch 1 dadurch gekennzeichnet, daß sie als orientierte dreidimensional vernetzte Substanzen mit flüssigkristalliner Struktur mit chiraler Phase Organosiloxane enthalten, bei denen die Zahl der polymerisierbaren Gruppen mindestens zwei beträgt.

3. Pigmente nach einem oder mehreren der Ansprüche 1 und 2, dadurch gekennzeichnet, daß sie als orientierte dreidimensional vernetzte Substanzen mit flüssigkristalliner Struktur mit chiraler Phase mindestens zwei Organosiloxane gemäß Anspruch zwei enthalten.

4. Pigmente nach einem oder mehreren der Ansprüche 1 bis 3, dadurch gekennzeichnet, daß sie als weiteres Pigment Ruß enthalten.

5. Verfahren zur Herstellung von Pigmenten nach einem oder mehreren der Ansprüche 1 bis 4, dadurch gekennzeichnet, daß dreidimensional vernetzbare Substanzen mit flüssigkristalliner Struktur mit chiraler Phase gegebenenfalls nach Zumischen weiterer Pigmente und/oder Farbstoffe orientiert werden, dreidimensional vernetzt werden und auf eine erwünschte Korngröße zerkleinert werden.

6. Verfahren nach Anspruch 5, dadurch gekennzeichnet, daß die Substanzen zum Orientieren auf eine Unterlage aufgebracht werden und von dieser vor dem Zerkleinern wieder abgelöst werden.

7. Zusammensetzung enthaltend mindestens ein Pigment nach einem oder mehreren der Ansprüche 1 bis 4, dadurch gekennzeichnet, daß sie zusätzlich noch mindestens eine Substanz aus der Gruppe Phenolharze, Aminharze, Alkydharze, Polyvinylacetatharze, Epoxidharze, Polyurethanharze, Polyethylenharze, Chlorkautschukharze, Cyclokautschukharze, chloriertes Polypropylen, Ketonharze, Acrylatharze, Melaminharze, Harnstoff-Formaldehyd-Harze, Phenol-Formaldehyd-Harze, oder mindestens eine Substanz aus der Gruppe Acrylnitril-Butadien-Styrol-Copolymere, Celluloseacetat, Celluloseacetobutyrat, Celluloseacetopropionat, Cellulosenitrat, Cellulosepropionat, Kunsthorn, Polyamid, Polycarbonat, Polyethylen, Polybutylenterephthalat, Polymethylmethacrylat, Polypropylen, Polystyrol, Polytetrafluorethylen, Polyvinylchlorid, Polyvinylidenchlorid, Polyurethan, Styrol- Acrylnitril-Copolymere, ungesättigte Polyesterharze enthält.

8. Verwendung von Pigmenten nach einem oder mehreren der Ansprüche 1 bis 4 zum Einfärben von Lacken, Kunststoffen, Faserrohstoffen, Druckfarben, oder kosmetischen Zubereitungen.

9. Verwendung von Pigmenten nach einem oder mehreren der Ansprüche 1 bis 4 als Sicherheitsmarkierung.

## Claims

1. Pigments having a thickness of 1 to 100 µm and a diameter of 1 to 10,000 µm and a colour which depends on the viewing angle, characterized in that they comprise oriented three-dimensionally crosslinked substances of liquid-crystalline structure having a chiral phase and, if desired, further dyes and pigments, the further dyes and pigments if present not serving as base for the oriented three-dimensionally crosslinked liquid-crystalline substances having a chiral phase.

2. Pigments according to Claim 1, characterized in that they contain, as the oriented three-dimensionally crosslinked substances of liquid-crystalline structure having a chiral phase, organosiloxanes in which the number of polymerizable groups is at least two.

3. Pigments according to one or more of Claims 1 and 2, characterized in that they contain, as the oriented three-dimensionally crosslinked substances of liquid-crystalline structure having a chiral phase, at least two organosiloxanes according to Claim 2.

4. Pigments according to one or more of Claims 1 to 3, characterized in that they contain carbon black as further pigment.

5. Process for preparing pigments according to one or more of Claims 1 to 4, characterized in that three-dimensionally crosslinkable substances of liquid-crystalline structure having a chiral phase are oriented, if appropriate after admixing further pigments and/or dyes, are crosslinked three-dimensionally and are comminuted to the desired particle size.

6. Process according to Claim 5, characterized in that the substances, for orientation, are applied to a backing and again removed therefrom before comminution.

7. Composition containing at least one pigment according to one or more of Claims 1 to 4, characterized in that additionally comprises at least one substance from the group comprising phenolic resins, amino resins, alkyd resins, polyvinyl acetate resins, epoxy resins, polyurethane resins, polyethylene resins, chlorinated rubber resins, cyclorubber resins, chlorinated polypropylene, ketone resins, acrylate resins, melamine resins, urea/formaldehyde resins and phenol/formaldehyde resins, or at least one substance from the group consisting of acrylonitrile/butadiene/styrene copolymers, cellulose acetate, cellulose acetobutyrate, cellulose acetopropionate, cellulose nitrate, cellulose propionate, casein plastics, polyamide, polycarbonate, polyethylene, polybutylene terephthalate, polymethyl methacrylate, polypropylene, polystyrene, polytetrafluoroethylene, polyvinyl chloride, polyvinylidene chloride, polyurethane, styrene/acrylonitrile copolymers and unsaturated polyester resins.

8. Use of pigments according to one or more of Claims 1 to 4 for the colouring of paints and coatings, plastics, fiber raw materials, printing inks, or cosmetic preparations.

9. Use of pigments according to one or more of Claims 1 to 4 as security marking.

## Revendications

1. Pigments ayant une épaisseur de 1 à 100 µm et un diamètre de 1 à 10 000 µm, présentant une couleur qui dépend de l'angle d'observation, caractérisés en ce qu'ils sont constitués de substances orientées à réticulation tridimensionnelle, ayant une structure mésomorphe à phase chirale, et éventuellement d'autres colorants et pigments, les autres colorants et pigments éventuellement présents ne servant pas de supports pour les substances mésomorphes à phase chirale orientées et à réticulation tridimensionnelle.

2. Pigments selon la revendication 1, caractérisés en ce qu'ils contiennent, en tant que substances orientées à réticulation tridimensionnelle ayant une structure mésomorphe à phase chirale, des organosiloxanes dans lesquels le nombre des groupes polymérisables est au moins égal à deux.

3. Pigments selon l'une ou plusieurs des revendications 1 et 2, caractérisés en ce qu'ils contiennent, en tant que substances orientées à réticulation tridimensionnelle, ayant une structure mésomorphe à phase chirale, au moins deux organosiloxanes selon la revendication 2.

4. Pigments selon l'une ou plusieurs des revendications 1 à 3, caractérisés en ce qu'ils contiennent, comme autre pigment, du noir de carbone.

5. Procédé pour préparer des pigments selon l'une ou plusieurs des revendications 1 à 4, caractérisé en ce qu'on oriente des substances pouvant subir une réticulation tridimensionnelle, ayant une structure mésomorphe à phase chirale, éventuellement après avoir ajouté d'autres pigments et/ou colorants, on les soumet à une réticulation tridimensionnelle et on le broie à la granulométrie voulue.

6. Procédé selon la revendication 5, caractérisé en ce que les substances destinées à être orientées sont appliquées sur un support et sont de nouveau détachées de ce dernier avant le broyage.

7. Composition contenant au moins un pigment selon l'une ou plusieurs des revendications 1 à 4, caractérisée en ce qu'elle contient en outre encore au moins une substance choisie parmi l'ensemble comprenant les résines phénoliques, les résines d'amines, les résines alkydes, les résines de poly(acétate de vinyle), les résines époxydes, les résines de polyuréthanne, les résines de polyéthylène, les résines de caoutchouc chloré, les résines de cyclocaoutchouc, le polypropylène chloré, les résines cétoniques, les résines d'acrylates, les résines de mélamine, les résines urée-formaldéhyde, les résines phénol-formaldéhyde ou au moins une substance choisie parmi l'ensemble comprenant les copolymères acrylonitrile-butadiène-styrène, l'acétate de cellulose, l'acétobutyrate de cellulose, l'acétopropionate de cellulose, le nitrate de cellulose, le propionate de cellulose, la corne artificielle, le polyamide, le polycarbonate, le polyéthylène, le poly(téréphtalate de butylène), le poly(méthacrylate de méthyle), le polypropylène, le polystyrène, le polytétrafluoréthylène, le poly(chlorure de vinyle), le poly(chlorure de vinylidène), le polyuréthanne, les copolymères styrène-acrylonitrile, les résines de polyester insaturées.

8. Utilisation de pigments selon l'une ou plusieurs des revendications 1 à 4 pour colorer dans la masse des vernis, des plastiques, des matières première fibreuses, des encres d'imprimerie ou des préparations cosmétiques.

9. Utilisation de pigments selon l'une ou plusieurs des revendications 1 à 4 comme marquage de sécurité.
